# EUROPÄISCHE PATENTSCHRIFT

(11) **EP 1 364 622 B1**
(45) Veröffentlichungstag und Bekanntmachung des Hinweises auf die Patenterteilung: **20.07.2005**
(21) Anmeldenummer: 03100604.2
(22) Anmeldetag: 11.03.2003
(51) Int. Cl.: A61B 17/70

(54) **Elastisches Stabilisiersystem für Wirbelsäulen**
Elastical system for stabilising the spine
Système élastique pour la stabilisation de la colonne vertébrale

(30) Priorität: 21.05.2002 CH 8532002
(43) Veröffentlichungstag der Anmeldung: 26.11.2003
(73) Patentinhaber: Spinelab GmbH, 3084 Wabern (CH)
(72) Erfinder: Freudiger, Stefan, 3047, Bremgarten b. Bern (CH)
(74) Vertreter: Scheuzger, Beat Otto

(56) Entgegenhaltungen:
- EP-A- 0 346 521
- EP-A- 0 669 109
- DE-A- 19 951 145
- FR-A- 2 718 946
- FR-A- 2 745 707
- FR-A- 2 799 949

## Beschreibung

Die vorliegende Erfindung betrifft ein Wirbelsäulenimplantat, bestehend aus einem Verbindungselement und mehreren Knochenschrauben mit je einer Aufnahme für dieses Verbindungselement. Das Implantat dient dem elastischen Stabilisieren einer Wirbelsäule einer Person mit schweren Rückenbeschwerden.

Der Stand der Technik liegt heute noch meistens in Stabilisiersystemen, welche ein knöchernes Verwachsen (Versteifung) betroffener Wirbelkörper zum Ziele haben. Elastische Systeme, welche die Wirbelsäulensegmente lediglich stützen und stabilisieren, nicht aber versteifen sollen, sind erst vereinzelt erschienen. Oft sind diese neueren elastischen Systeme noch mit einem erheblichen Implantationsaufwand verbunden. Aus Gründen der Herstellkosten, der Operationszeit und der Sicherheit in der Handhabung sollen künftige Wirbelsäulenstabilisiersysteme so einfach wie möglich sein.

Die Vorteile von elastischen Stabilisierungen werden zunehmend bekannter, vor allem bei jungen Patienten, so dass verschiedene Erfinder solche Systeme entwickelt und offengelegt haben. Diese Erfindungen bergen aber Nachteile in sich, welche anhand folgender Beispiele erläutert werden.

Die Erfindung gemäss der Patentschrift EP 0498 709 B1 (Graf) möchte zwar elastisch stabilisieren, weist jedoch den Nachteil auf, dass das System nur in Flexion (Zug), nicht aber in Extension (Druck) wirkt. Die Stabilisierung wird durch mindestens zwei einzelne textile Schlingen erzeugt, die gegenseitig versetzt sind.

Die Erfindung gemäss der Patentanmeldung WO 93/20771 (Mazel) möchte Wirbelkörper auch mit flexiblen paarweisen Längsstäbchen verbinden. Diese Erfindung weist jedoch die Nachteile auf, dass die Längsstäbchen kaum Knickfestigkeit haben und damit kaum Druckkräfte übertragen können, dass die Biegsamkeit der paarweise angeordneten Stäbchen nicht in alle Richtungen gleich sein kann und dass die Kraftübertragung in die Knochenschrauben mit zahlreichen Einzelteilen erfolgen muss.

Die Erfindung gemäss der Patentschrift EP 0516 567 B1 (Navas) will Stossdämpfer zwischen die Wirbelkörper einbringen. Diese Erfindung weist jedoch die Nachteile auf, dass diese Stossdämpfer nicht längsverstellbar sind und der Kunststoff durch eine enge Stelle (Hals) geführt wird, was eine erhebliche Verminderung der Festigkeit zur Folge hat.

Die Erfindung gemäss der Patentschrift US 5,282,863 A (Burton) will auch flexibel stabilisieren. Diese Erfindung weist jedoch die Nachteile auf, dass das System zu breit ist und posterior nur gesetzt werden kann, wenn die Pedikel entfernt sind, dass es nur für ein Segment verwendet werden kann, dass es zum Beispiel nicht durch drei hintereinander liegende und versetzte Schrauben geführt werden kann, dass die Bohrung im Verbindungselement eine erhebliche Schwächung zur Folge hat und dass der ovale Querschnitt des Verbindungselementes nur eine minimale Schubsteifigkeit und minimale Knickfestigkeit in anteriorer / posteriorer Richtung aufweist. Weiter muss zur Befestigung des Verbindungselements an der Pedikelschraube eine Verschlusskappe verwendet werden, was bei der Operation nachteilig ist.

Die Erfindung gemäss der Patentanmeldung EP 0 667 127 A1 (Sanders) will eine gewisse Elastizität durch eine metallische Verbindung erreichen, indem die Gestaltung des Verbindungsteiles lokale Biegung zulässt. Diese Erfindung weist jedoch den Nachteil auf, dass die Verbindungsteile nicht längsverstellbar sind und nicht mehrsegmental eingesetzt werden können.

Die Erfindung gemäss der Patentschrift EP 0669 109 B1 (Baumgartner/Freudiger/Dubois) will benachbarte Wirbelsegmente ebenfalls elastisch stabilisieren, indem für Zugkräfte ein Band und für Druckkräfte ein Kunststoffkissen eingesetzt werden. Diese Erfindung weist jedoch die Nachteile auf, dass das System ein teures Band enthält, beliebige Kissenhöhen nur durch eine Mehrzahl von Standard-Kissen erreicht werden kann, die Variation der Vorspannung auf das Band zu biomechanisch nicht reproduzierbaren Verhältnissen führt und die Implantation relativ aufwendig ist und lange dauert.

Zudem ist aus der FR-A-2 718 946 (Basis für dem Oberbegriff des Anspruchs 1.) ein osteosynthetisches Fixiersystem bekannt, bei welchem als Stabilisierungselement ein spiralfederförmiges Glied aus Metall eingesetzt wird. Dieses spiralfederförmige Glied ist, wenn es zum Einlegen in die Knochenschrauben ohne grossen Kraftaufwand gebogen werden soll, zu wenig druck- und zugstabil, zudem ist die Schubsteifigkeit viel zu gering. Ferner muss dieses spiralfederförmige Glied mit den Knochenschrauben kraftschlüssig mit hoher Pressung verspannt werden. Minimale Gleitbewegungen sind innerhalb dieser Verbindung nicht zu vermeiden, was einen Abrieb von Material mit sich bringt, der verheerende Folgen auf den Patienten haben kann.

Der vorliegenden Erfindung liegen demnach die Aufgaben zugrunde, sowohl Zug- wie auch Druckkräfte zwischen benachbarten Wirbelkörpern mit ein und demselben Verbindungselement zu übertragen und durch Knochenschrauben, vorzugsweise in die Pedikel eingesetzt, durchführbar oder in dieselben einlegbar zu sein, welche naturgemäss nicht auf einer Achse liegen.

Die Losung dieser Aufgabe zeichnet sich dadurch aus, dass ein elastisches Verbindungselement verwendet wird, welches aufgrund der örtlichen, maximal zulässigen, Querschnitten ausreichend Schub-, Zug-, Druck- und Knickfestigkeit erzielt, um die zu erwartenden Kräfte dauerhaft und sicher zu übertragen.

Gegenstand der Erfindung ist demzufolge ein Wirbelsäulenimplantat, bestehend aus einem Verbindungselement und mehreren Knochenschrauben mit je einer Aufnahme für dieses Verbindungselement, das um jede Achse seines Querschnittes elastisch biegsam ist, derart, dass es durch die Aufnahmen mehrerer Schraubenköpfe hintereinander durchgeführt oder eingelegt werden kann, auch wenn sich diese nicht auf ein und derselben Achse befinden dadurch gekennzeichnet ist, dass das Verbindungselement ein Stab ist, der aus einem elastischen biokompatiblen Kunststoff besteht, und dass das Verbindungselement und die Aufnahme im Schraubenkopf ganz oder teilweise eine strukturierte Oberfläche aufweisen, derart, dass die Struktur der Aufnahme in die Struktur des Verbindungselements formschlüssig eingreift und im zusammengefügten Zustand ein Verschieben verhindert.

Das um jede Achse seines Querschnittes elastisch biegsame Verbindungselement besteht aus einem elastisch biegsamen biokompatiblen Kunststoff. Ein solcher Kunststoff ist ein Polymer, das aus gleichen oder verschieden Bausteinen aufgebaut sein kann und die gewünschten mechanischen und chemischen Eigenschaften besitzt, zum Beispiel ein Material auf Basis von Polyurethan, wie aromatische Polycarbonat-Polyurethane (geeignete Handelsprodukte sind beispielsweise: BIONATE ® von Polymer Technology Group, 2810 7th Street, Berkeley, California 94710 USA und ChronoFlex®C von CardioTech International Inc., 78E Olympia Ave., Woburn, MA 01801-2057, USA). Das erfindungsgemäss vorgeschlagene Verbindungselement besitzt eine ausreichende Biegeelastizität um alle Achsen seines Querschnittes, derart, dass das Einführen desselben auch in Aufnahmen von Schraubenköpfen ermöglicht wird, welche nicht auf einer Achse, sondern auf einer willkürlich verlaufenden Linie liegen, oder aufgrund unterschiedlicher Wirbelkörperanordnungen naturgemäss in verschiedene Richtungen versetzt sind.

Zwecks Fixierung der Verbindungselemente mit den Aufnahmen können ihre Oberflächen, die bei der Fixierung miteinander in Kontakt kommen mit einer ineinander eingreifbaren Oberflächenstruktur versehen sein, derart, dass wenn ein Verbindungselement in eine Aufnahme eingeklinkt ist, ein gegenseitiges Verschieben nicht mehr möglich ist. Diese Oberflächenstruktur ist beispielsweise eine geeignete Rillenstruktur mit Rillen in Querrichtung zum Verbindungselement. Die Rillenstruktur kann auch eine Gewindestruktur sein, die ein Einschrauben ermöglicht. Es können auch andere ineinander eingreifbare Oberflächenstrukturen ausgewählt werden, wie beispielsweise eine Noppen/Vertiefungsstruktur. Bei der Verwendung einer Rillenstruktur in Querrichtung zur Längsachse des Verbindungselementes, kann die Rillenstruktur in der Aufnahme so ausgestaltet sein, dass ein Eingreifen der Rillenstruktur des Verbindungselementes in eine erweiterte Rillenstruktur in der Einschuböffnung der Aufnahme möglich ist. Dadurch kann eine Vor-Fixierung erzielt werden, damit der Chirurg vor dem Einklinken des Verbindungselements in die Aufnahme seine anatomisch korrekte Position kontrollieren kann.

Das Verbindungselement mit dem stabilisierenden Effekt kann einen in Richtung der Stabachse veränderten Querschnitt aufweisen, damit es eine positionsabhängige veränderliche Steifigkeit aufweist, was ihm einen lokal angepassten Stabilisierungseffekt verleiht. Dadurch kann der Stabilisierungseffekt benachbarter Wirbelkörper eingestellt werden, bis hin zur lokalen Versteifung mit graduellen Übergängen. Hierzu kann das Verbindungselement die Form eines hohlen Stabes mit variierter Wandstärke sein. Wenn im nachstehenden Text im Zusammenhang mit dem Verbindungselement von "originalem Querschnitt" gesprochen wird, bedeutet dies, dass der Querschnitt im Wesentlichen dem ursprünglichen, vor dem Einsetzen des Elementes vorgelegenen Querschnitt entspricht, was nicht ausschliesst, dass Abweichungen vorkommen können, z.B. durch Zusammendrücken bzw. durch Vorspannung durch das am Schraubenkopf angeordnete Befestigungsmittel.

Die Aufnahmen, die in die Köpfe der Knochenschrauben integriert sind, besitzen vorzugsweise eine C-förmige Form, in welche das elastische Verbindungselement durch den Chirurgen in der korrekten Position eingeklinkt werden kann. Hierzu kann die Aufnahme derart ausgestaltet sein, dass sie ebenfalls in einem bestimmten Ausmass elastisch ist. Dadurch wird bei der Operation eine Befestigung ohne zusätzliche kleine Implantatteile ermöglicht.

Das erfindungsgemässe Wirbelsäulenimplantat ist in der Lage, durch degenerative oder iatrogene Vorgänge instabil und dadurch schmerzhaft gewordene Wirbelsäulen zu stabilisieren und damit Schmerzen zu verringern oder ganz zu vermeiden. Der Vorteil einer elastischen Stabilisierung liegt vor allem darin, dass einzelne Wirbelkörper nicht mehr wie früher versteift werden müssen, was in vielen Fallen zu Folgeschäden an Nachbarsegmenten geführt hat. Der besondere Vorteil der vorliegenden Erfindung liegt in den geringen Herstellkosten sowie in der einfachen und sicheren Implantationstechnik des Systems.

Die nachstehende Figurenliste gibt einen Überblick über die beiliegenden Zeichnungen.

Es zeigen schematisch:
Fig. 1 ein Wirbelsäulensegment mit mehreren Wirbelkörpern mit linken und rechten Pedikelschrauben und einem linken und rechten elastisch biegsamen Verbindungsstab.
Fig. 1a eine Teilansicht eines Wirbelsäulensegmentes, jedoch mit einer alternativen Aufnahme im Kopf der Pedikelschrauben.
Fig. 2 beispielsweise drei Pedikelschrauben mit gegeneinander versetzten Achsen und dem eingeführten elastisch biegsamen Verbindungsstab.
Fig. 2a eine Teilansicht von Fig. 2 mit einer alternativen Pedikelschraube.
Fig. 3 eine Teilansicht eines elastisch biegsamer Verbindungsstab mit gerillter Oberfläche.
Fig. 4 und 4a Ansichten einer Pedikelschraube mit einer gerillten Aufnahme im Kopf.
Fig. 5 und 5a Ansichten einer Pedikelschraube mit einer gerillten Aufnahme im Kopf, wo die Rillenstruktur in den Aufnahmeschlitz und die darüber hinausragende Anschrägung ausgedehnt ist.
Fig. 6a die Aufnahme mit Schlitz im Schraubenkopf und den Stab ausserhalb der Aufnahme. Fig. 6b den elastisch zusammengedrückten Stab beim Einführen in die Aufnahme mit Schlitz im Schraubenkopf. Fig. 6c den elastischen Stab in seinem originalen Querschnitt in der Aufnahme mit Schlitz.
Fig. 7a die Aufnahme mit Schlitz im Schraubenkopf und den Stab mit einer abgeflachten Seite ausserhalb der Aufnahme. Fig. 7b den elastisch zusammengedrückten Stab mit einer abgeflachten Seite beim Einführen in die Aufnahme mit Schlitz im Schraubenkopf. Fig. 7c den elastischen Stab mit einer abgeflachten Seite in seinem originalen Querschnitt gedreht in der Aufnahme mit Schlitz.
Fig. 8a die Aufnahme mit Schlitz im Schraubenkopf und den Stab mit zwei parallel abgeflachten Seiten ausserhalb der Aufnahme. Fig. 8b den elastisch zusammengedrückten Stab mit zwei parallel abgeflachten Seiten beim Einführen in die Aufnahme mit Schlitz im Schraubenkopf. Fig. 8c den elastischen Stab mit zwei parallel abgeflachten Seiten in seinem originalen Querschnitt gedreht in der Aufnahme mit Schlitz.
Fig. 9a die Aufnahme mit Schlitz im Schraubenkopf und den Stab mit einem Hohlraum im Zentrum ausserhalb der Aufnahme. Fig. 9b den elastisch zusammengedrückten Stab mit einem Hohlraum im Zentrum beim Einführen in die Aufnahme mit Schlitz im Schraubenkopf. Fig. 9c den elastischen Stab mit einem Hohlraum im Zentrum in seinem originalen Querschnitt mit einem Füllstück im Hohlraum in der Aufnahme mit Schlitz.
Fig. 10a die Aufnahme mit Schlitz mit einer Klemmvorrichtung. Fig. 10b den elastischen Stab geklemmt in der Aufnahme mit Schlitz.
Fig. 11 die Aufnahme mit Schlitz mit einer Hakenvorrichtung und einem eingehakten Keil.
Fig. 12a ein elastisch biegsamer Verbindungsstab mit gerillter, mit einer Steigung versehenen Oberfläche. Fig. 12b die gerillte, mit derselben Steigung versehenen Aufnahme im Kopf der Pedikelschraube.

Im Folgenden wird die vorliegende Erfindung anhand der beiliegenden Zeichnungen, welche lediglich Ausführungsbeispiele darstellen, näher erläutert.

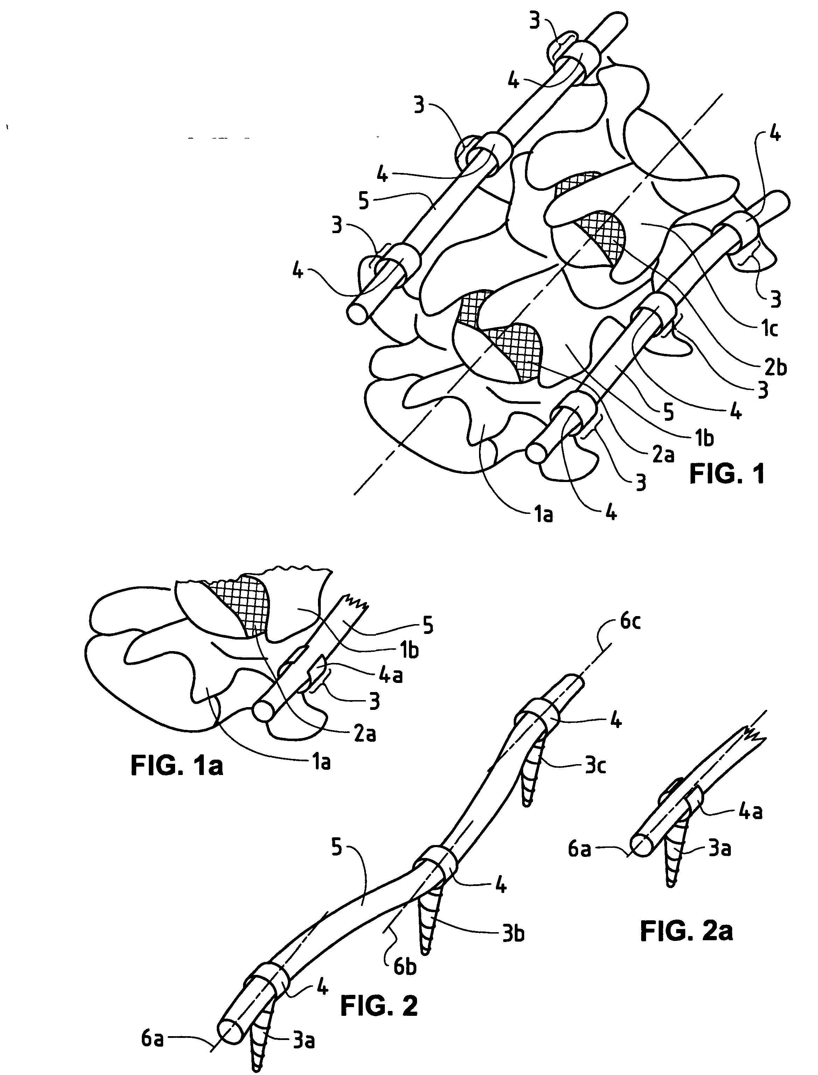

Fig. 1 stellt ein Wirbelsäulensegment dar, mit drei Wirbelkörpern 1a, 1b, 1c und zwei dazwischen liegenden Bandscheiben 2a, 2b. Pro Wirbelkörper ist jeweils links und rechts eine Pedikelschraube 3 mit je einer Aufnahme 4 eingesetzt, in welche links und rechts je ein elastisch biegsames stabförmiges Verbindungselement 5 befestigt ist. Die Verbindungselemente 5 sind in den Aufnahmen 4 gelagert und dienen der flexiblen Stabilisierung der Wirbelkörper.
Fig. 1a ist eine Teilansicht eines analogen Wirbelsäulensegmentes mit einer Pedikelschraube 3 mit einer offenen Aufnahme 4a für die Lagerung eines Verbindungselementes 5.
Fig. 2 zeigt drei Pedikelschrauben 3a, 3b, 3c mit gegeneinander versetzten Achsen (6a, 6b, 6c) ihrer Aufnahmen im Kopf und dem eingeführten elastisch biegsamen stabförmigen Verbindungselement 5.
Fig. 2a zeigt eine Teilansicht entsprechend Fig. 2, jedoch mit einer offenen Aufnahme 4a für das Verbindungselement 5.

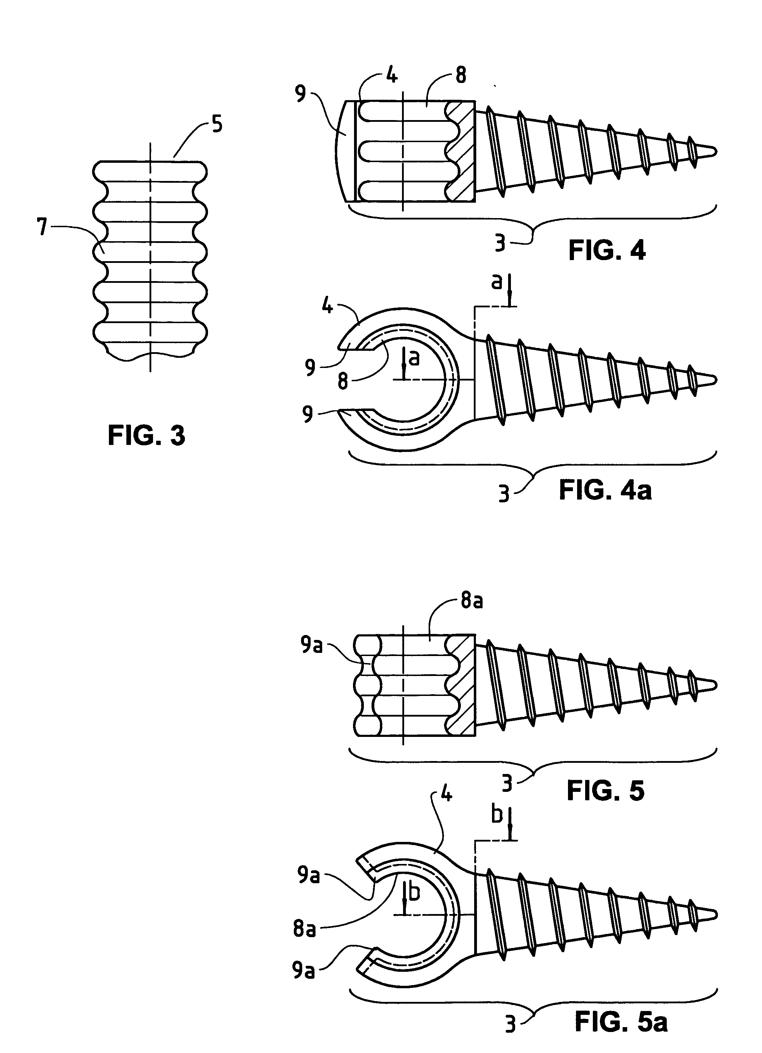

Fig. 3 zeigt eine Teilansicht eines elastisch biegsamen stabförmigen Verbindungselements 5 mit gerillter Oberfläche 7. Die Rillung dient dem
formschlüssigen Eingreifen in eine entsprechend ausgestaltete Aufnahme einer Pedikelschraube.
Fig. 4 zeigt eine Seitenansicht einer Pedikelschraube 3a, deren Kopf als Teilschnitt (Ebene a-a in Fig. 4a) dargestellt ist. Der Kopf ist als Aufnahme 4 ausgestaltet, die eine entsprechend dem Verbindungsstab innere gerillte Oberfläche mit Rippen 8 besitzt. In der Aufnahmeöffnung sind beidseitig Anschrägungen 9 vorgesehen, welche das Einsetzen eines Stabilisierungselementes erleichtern. Fig. 4a zeigt die gleiche Pedikelschraube, die jedoch bezüglich ihrer Längsachse, die in der Blattebene liegt, um 90° gedreht ist. Sichtbar ist hier die Öffnung der Aufnahme 4 mit den beiden Anschrägungen 9 und einer Rippe 8 der inneren gerillten Oberfläche.
Fig. 5 ist eine Seitenansicht einer weiteren Ausführungsform einer Pedikelschraube 3, deren Kopf, der auch als Teilschnitt (entlang der Linie b-b von Fig. 5a) dargestellt ist, als modifizierte Aufnahme 4 ausgestaltet ist. Die Aufnahme besitzt im Aufnahmeschlitz eine gerillte Oberfläche mit Rippen 8a, die sich bis in die Anschrägung 9a ausdehnen. Durch diese Ausgestaltung der Aufnahme kann ein entsprechend gerilltes Verbindungselement vor dem Einrasten in die Aufnahme verschiebungsfrei vorpositioniert werden, damit der Chirurg die korrekte Position kontrollieren kann. Fig. 5a zeigt die gleiche Pedikelschraube, die jedoch bezüglich ihrer Längsachse, die in der Blattebene liegt, um 90° gedreht ist. Sichtbar ist hier die Öffnung der Aufnahme 4 mit den beiden Anschrägungen 9a und einer Rippe 8a der inneren gerillten Oberfläche. Sichtbar ist hier die Ausdehnung der gerillten Oberfläche auf die Anschrägung 9a.

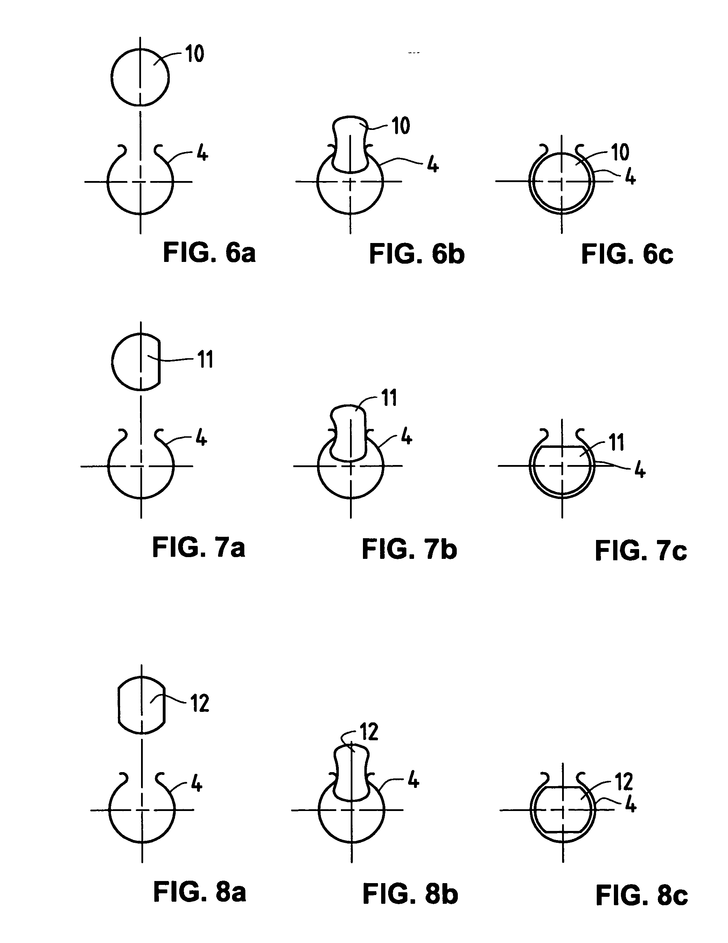

Fig. 6a zeigt schematisch im Schnitt eine Aufnahme 4 mit Schlitz im Schraubenkopf einer Pedikelschraube und ein Verbindungselement 10, das sich noch ausserhalb der Aufnahme befindet. Fig. 6b zeigt das elastisch zusammengedrückte Verbindungselement 10 beim Einführen in den Schlitz der Aufnahme 4 im Schraubenkopf. Fig. 4c zeigt im Schnitt das elastischen Verbindungselement 10 wiederum in seinem originalen Querschnitt in die Aufnahme 4 mit Schlitz im Schraubenkopf eingebracht. Hier wird er mit Hilfe der gerillten Oberflächen formschlüssig verankert.
Fig. 7a zeigt im Schnitt den als Aufnahme 4 mit Schlitz ausgestalteten Schraubenkopf und das stabförmige Verbindungselement 11 mit einer abgeflachten Seite noch ausserhalb der Aufnahme 4. Fig. 7b zeigt das elastisch zusammengedrückte Verbindungselement 11 mit einer abgeflachten Seite beim Einführen in die Aufnahme mit Schlitz im Schraubenkopf. Fig. 7c zeigt das elastische Verbindungselement 11 mit einer abgeflachten Seite wiederum in seinem originalen Querschnitt in die Aufnahme 4 mit Schlitz im Schraubenkopf eingebracht, um 90° gedreht und mit Hilfe der gerillten Oberflächen formschlüssig verankert.
Fig. 8a zeigt im Schnitt die Aufnahme 4 mit Schlitz im Schraubenkopf und das Verbindungselement 12 mit zwei parallel abgeflachten Seiten noch ausserhalb der Aufnahme 4. Fig. 8b zeigt das elastisch zusammen gedrückte Verbindungselement mit zwei parallel abgeflachten Seiten 12 beim Einführen in die Aufnahme 4 mit Schlitz im Schraubenkopf. Fig. 8c zeigt das elastische Verbindungselement 12 mit zwei parallel abgeflachten Seiten wiederum in seinem originalen Querschnitt in die Aufnahme 4 mit Schlitz im Schraubenkopf eingebracht, um 90° gedreht und mit Hilfe der gerillten Oberflächen formschlüssig verankert.

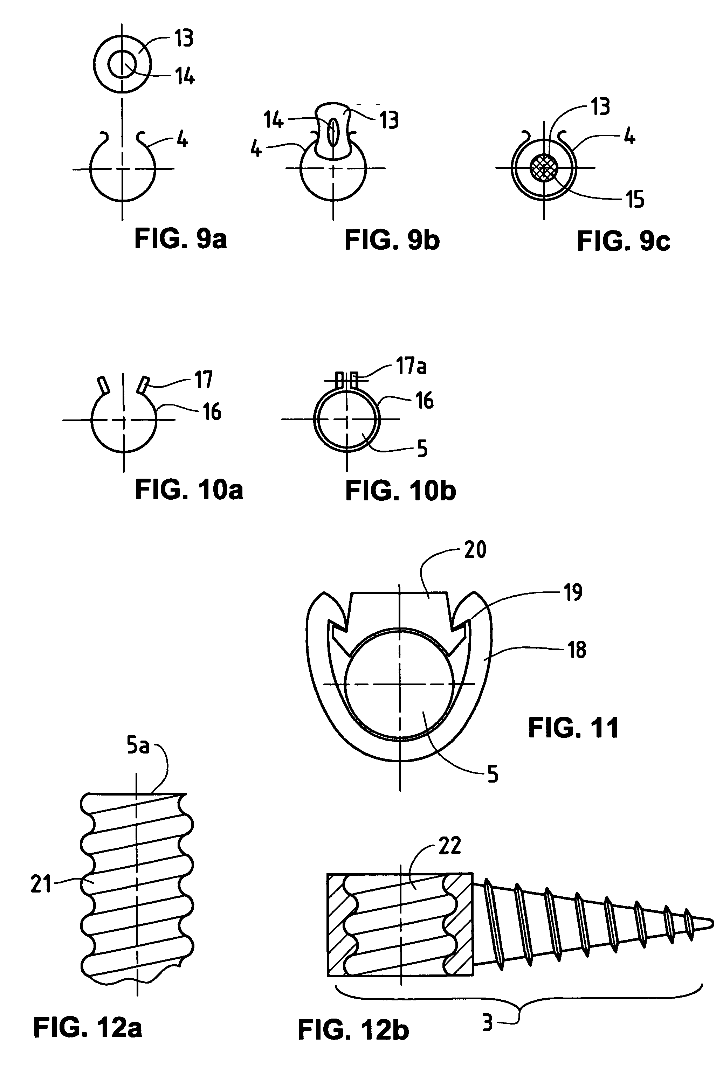

Fig. 9a zeigt im Schnitt die Aufnahme 4 mit Schlitz im Schraubenkopf und ein Verbindungselement 13 mit einem Hohlraum 14 im Zentrum noch ausserhalb der Aufnahme 4. Fig. 9b zeigt das elastisch zusammengedrückte Verbindungselement 13 mit einem Hohlraum 14 im Zentrum beim Einführen in die Aufnahme 4 mit Schlitz im Schraubenkopf. Der Hohlraum 14 im Zentrum des Verbindungselementes 13 erleichtert hierbei das elastische Zusammendrücken des Verbindungselementes zum einfacheren Einführen. Fig. 9c zeigt das elastische Verbindungselement 13 mit einem Hohlraum 14 im Zentrum wiederum in seinem originalen Querschnitt in die Aufnahme 4 mit Schlitz im Schraubenkopf eingebracht, aber nunmehr mit einem den Hohlraum ausfüllenden (optionalen) Zapfen 15 versehen und mit Hilfe der gerillten Oberflächen, die sich in der Aufnahme 4 und auf dem Verbindungselement 13 befinden, formschlüssig verankert.
Fig. 10a zeigt im Schnitt eine weitere Ausführungsform einer Aufnahme 16 mit Schlitz im Schraubenkopf versehen mit einer Klemmvorrichtung 17. Fig. 10b zeigt die Aufnahme 16 mit Schlitz im Schraubenkopf, einem eingeführten Verbindungselement 5 in seinem geklemmten Querschnitt und mit zusammengezogener Klemmvorrichtung 17.
Fig. 11 zeigt eine weitere Ausführungsform einer Aufnahme18 mit Schlitz im Schraubenkopf versehen mit einer Hakenvorrichtung 19 und einem unter Vorspannung eingehakten Keil 20 zum Festhalten des Verbindungselementes 5.
Fig. 12a zeigt eine Teilansicht eines elastisch biegsamen stabförmigen Verbindungelements 5a mit gerillter, mit einer Steigung 21 versehenen Oberfläche und Fig. 12b zeigt die gerillte, mit derselben Steigung 22 versehenen Aufnahme im Kopf der Pedikelschraube 3. Die Rillen mit Steigung bewirken, dass sich der Stab wie eine Schraube und die Aufnahme im Schraubenkopf wie eine Mutter verhalten, so dass der Stab durch Drehung um die Längsachse in die Schraubenköpfe eingeschraubt und durchgeführt werden kann.

## Patentansprüche

1. Wirbelsäulenimplantat, bestehend aus einem Verbindungselement (5) und mehreren Knochenschrauben (3) mit je einer Aufnahme (4) für dieses Verbindungselement, das um jede Achse seines Querschnittes elastisch biegsam ist, derart, dass es durch die Aufnahmen (4) mehrerer Schraubenköpfe hintereinander durchgeführt oder eingelegt werden kann, auch wenn sich diese nicht auf ein und derselben Achse (6a, 6b, 6c) befinden, **dadurch gekennzeichnet, dass** das Verbindungselement (5) ein Stab ist, der aus einem elastischen biokompatiblen Kunststoff besteht, und dass das Verbindungselement (5) und die Aufnahme (4) im Schraubenkopf ganz oder teilweise eine strukturierte Oberfläche (7, 8) aufweisen, derart, dass die Struktur der Aufnahme (4) in die Struktur des Verbindungselements (5) formschlüssig eingreift und im zusammengefügten Zustand ein Verschieben verhindert.

2. Wirbelsäulenimplantat nach Anspruch 1, **dadurch gekennzeichnet, dass** der biokompatible Kunststoff aus einer oder mehreren Arten von Monomerbausteinen aufgebaut sein kann.

3. Wirbelsäulenimplantat nach Anspruch 2, **dadurch gekennzeichnet, dass** der biokompatible Kunststoff ein Kunststoff auf Basis von Polyurethan ist.

4. Wirbelsäulenimplantat nach einem der Ansprüche 1 bis 3, **dadurch gekennzeichnet, dass** das elastische Verbindungselement eine Struktur mit einem oder mehreren Hohlräumen besitzt.

5. Wirbelsäulenimplantat nach Anspruch 4, **dadurch gekennzeichnet dass** das elastische Verbindungselement einen rohrförmigen Querschnitt besitzt, mit einer entlang dem Verbindungselement variablen Wandstärke, die entsprechend der Position-dem Verbindungselement die gewünschte variable Steifigkeit verleiht.

6. Wirbelsäulenimplantat nach einem der Ansprüche 1 bis 5, **dadurch gekennzeichnet, dass** die strukturierte Oberflächen eine im Wesentlichen in Querrichtung zur Längsachse des Verbindungselements gerillte Struktur aufweist.

7. Wirbelsäulenimplantat nach einem der Ansprüche 1 bis 6, **dadurch gekennzeichnet, dass** die Aufnahme (4) des Schraubenkopfes einen Schlitz aufweist, so dass das elastische Verbindungselement unter elastischer Deformation in die Aufnahme eingesetzt und verankert werden kann.

8. Wirbelsäulenimplantat nach Anspruch 7, **dadurch gekennzeichnet, dass** die Rillenstruktur (8a) der Aufnahme (4) des Schraubenkopfes in die Anschrägungen (9a) des Schlitzes weitergeführt wird, so dass das elastische Verbindungselement zur Überprüfung des korrekten Abstandes der Wirbelkörper vorpositioniert werden kann.

9. Wirbelsäulenimplantat nach einem der Ansprüche 1 bis 8, **dadurch gekennzeichnet, dass** das Verbindungselement (11) einen runden Querschnitt mit einer flachen Seite aufweist, so dass das Verbindungselement mit einer reduzierten lichten Weite in die Aufnahme (4) eingebracht und anschliessend durch eine Drehung verankert werden kann.

10. Wirbelsäulenimplantat nach einem der Ansprüche 1 bis 9, **dadurch gekennzeichnet, dass** das Verbindungselement einen runden Querschnitt mit zwei parallelen flachen Seiten aufweist (12), so dass das Verbindungselement mit einer reduzierten lichten Weite in die Aufnahme eingebracht und anschliessend durch eine Drehung verankert werden kann.

11. Wirbelsäulenimplantat nach einem der Ansprüche 1 bis 10, **dadurch gekennzeichnet, dass** das Verbindungselement (13) einen Hohlraum (14) um die Längsachse aufweist, welcher die elastische Deformation zum Einbringen in die Aufnahme erleichtert.

12. Wirbelsäulenimplantat nach Anspruch 11, **dadurch gekennzeichnet, dass** es einen Zapfen (15) umfasst, der nach dem Einbringen des Verbindungselementes (13) in die Aufnahme (4) in den Hohlraum (14) eingeschoben werden kann.

13. Wirbelsäulenimplantat nach einem der Ansprüche 1 bis 12, **dadurch gekennzeichnet, dass** die Aufnahme des Schraubenkopfes klemmbar ist (17) und das Verbindungselement (5) nach dem Einführen geklemmt werden kann (17a).

14. Wirbelsäulenimplantat nach einem der Ansprüche 1 bis 12, **dadurch gekennzeichnet, dass** die Aufnahme des Schraubenkopfes eine Hakenvorrichtung (19) aufweist, in welche, unter Vorspannung des Verbindungselementes (5), ein Keil (20) eingehakt werden kann.

15. Wirbelsäulenimplantat nach einem der Ansprüche 6 bis 14, **dadurch gekennzeichnet, dass** die Rillen am Verbindungselement eine Steigung aufweisen (21), die Aufnahme am Schraubenkopf dieselben Rillen mit Steigung (22) aufweist und somit wie eine Mutter wirkt, so dass das Verbindungselement durch Drehung um die Langsachse durch die Schraubenkopfe eingeschraubt und durchgeführt werden kann.

## Claims

1. Vertebral column implant, composed of a connecting element (5) and a multiplicity of bone screws (3), each having a seat (4) for this connecting element, which is bendable elastically about every axis of its cross-section in such a way that it can be passed through or inserted in the seats (4) of a number of screw heads one behind the other even when the latter are not situated on one and the same axis (6a, 6b, 6c), **characterised in that** the connecting element (5) is a rod that is made of an elastic biocompatible plastic, and **in that** the connecting element (5) and the seat (4) in the screw head have wholly or in part a structured surface (7, 8), in such a way that the structure of the seat (4) engages in a form-fitting way the structure of the connecting element (5) and prevents a shifting in assembled condition.

2. Vertebral column implant according to claim 1, **characterised in that** the biocompatible plastic may be composed of one or more kinds of monomer components.

3. Vertebral column implant according to claim 2, **characterised in that** the biocompatible plastic is a plastic on the basis of polyurethane.

4. Vertebral column implant according to one of the claims 1 to 3, **characterised in that** the elastic connecting element has a structure with one or more hollow spaces.

5. Vertebral column implant according to claim 4, **characterised in that** the elastic connecting element has a tubular cross-section with a wall thickness variable along the connecting element, which wall thickness confers the desired variable stiffness upon the connecting element according to the position.

6. Vertebral column implant according to one of the claims 1 to 5, **characterised in that** the structured surfaces have a structure grooved substantially at right angles to the longitudinal axis of the connecting element.

7. Vertebral column implant according to one of the claims 1 to 6, **characterised in that** the seat (4) of the screw head has a slot, so that the elastic connecting element can be inserted and anchored in the seat with elastic deformation.

8. Vertebral column implant according to claim 7, **characterised in that** the grooved structure (8a) of the seat (4) of the screw head is continued into the bevels (9a) of the slot so that the elastic connecting element can be pre-positioned for verifying the correct spacing of the vertebrae.

9. Vertebral column implant according to one of the claims 1 to 8, **characterised in that** the connecting element (11) has a round cross-section with a flat side so that the connecting element can be introduced into the seat (4) with a reduced inside width and subsequently anchored by means of a rotation.

10. Vertebral column implant according to one of the claims 1 to 9, **characterised in that** the connecting element has a round cross-section with two parallel flat sides (12), so that the connecting element, with a reduced inside width, can be introduced into the seat and subsequently anchored by means of a rotation.

11. Vertebral column implant according to one of the claims 1 to 10, **characterised in that** the connecting element (13) has a hollow space (14) about the longitudinal axis, which hollow space facilitates the elastic deformation for introduction into the seat.

12. Vertebral column implant according to claim 11, **characterised in that** it comprises a plug (15) which can be pushed into the hollow space (14) after introduction of the connecting element (13) into the seat (4).

13. Vertebral column implant according to one of the claims 1 to 12, **characterised in that** the seat of the screw head is clampable (17), and the connecting element (5) can be clamped (17a) after introduction.

14. Vertebral column implant according to one of the claims 1 to 12, **characterised in that** the seat of the screw head has a hook device (19) into which a wedge (20) can be hooked with prestressing of the connecting element (5).

15. Vertebral column implant according to one of the claims 6 to 14, **characterised in that** the grooves on the connecting element have a pitch (21), the seat on the screw head has the same grooves with pitch (22) and thus acts like a nut, so that the connecting element can be screwed in and passed through the screw heads by rotation about the longitudinal axis.

## Revendications

1. Implant de la colonne vertébrale, se composant d'un élément de connexion (5) et de plusieurs vis osseuses (3) avec chacune un logement (4) pour cet élément de liaison qui est souple de manière élastique autour de chaque axe de sa section transversale, de sorte qu'il peut être inséré et guidé à travers les logements (4) de plusieurs têtes de vis les unes derrière les autres, même si ces dernières ne se trouvent pas sur un seul et même axe (6a, 6b, 6c), **caractérisé en ce que** l'élément de liaison (5) est une barre qui se compose de matière plastique élastique biocompatible, et **en ce que** l'élément de liaison (5) et le logement (4) présentent dans la tête de vis entièrement ou partiellement une surface structurée (7, 8), de sorte que la structure du logement (4) s'engage par adhérence des formes dans la structure de l'élément de liaison (5) et empêche un déplacement à l'état assemblé.

2. Implant de la colonne vertébrale selon la revendication 1, **caractérisé en ce que** la matière synthétique biocompatible peut être composée de un ou de plusieurs composants monomères.

3. Implant de la colonne vertébrale selon la revendication 2, **caractérisé en ce que** la matière synthétique biocompatible est une matière plastique à base de polyuréthane.

4. Implant de la colonne vertébrale selon l'une des revendications 1 à 3, **caractérisé en ce que** l'élément de liaison élastique possède une structure avec une ou plusieurs cavités.

5. Implant de la colonne vertébrale selon la revendication 4, **caractérisé en ce que** l'élément de liaison élastique possède une section transversale tubulaire, avec une épaisseur de paroi variable le long de l'élément de liaison, qui confère à l'élément de liaison la rigidité variable souhaitée en fonction de la position.

6. Implant de la colonne vertébrale selon l'une des revendications 1 à 5, **caractérisé en ce que** les surfaces structurées présentent une structure nervurée essentiellement dans le sens transversal à l'axe longitudinal de l'élément de liaison.

7. Implant de la colonne vertébrale selon l'une des revendications 1 à 6, **caractérisé en ce que** le logement (4) de la tête de vis présente une fente de sorte que l'élément de liaison élastique peut être inséré et ancré dans le logement par déformation élastique.

8. Implant de la colonne vertébrale selon la revendication 7, **caractérisé en ce que** la structure nervurée (8a) du logement (4) de la tête de vis est guidée dans les biseaux (9a) de la fente de sorte que l'élément de liaison élastique peut être prépositionné pour la vérification de la bonne distance des corps vertébraux.

9. Implant de la colonne vertébrale selon l'une des revendications 1 à 8, **caractérisé en ce que** l'élément de liaison (11) présente une section transversale ronde avec une face plate de sorte que l'élément de liaison peut être introduit dans le logement (4) avec un diamètre intérieur et peut être ancré ensuite par une rotation.

10. Implant de la colonne vertébrale selon l'une des revendications 1 à 8, **caractérisé en ce que** l'élément de liaison présente une section transversale ronde avec deux faces plates parallèles (12) de sorte que l'élément de liaison peut être introduit dans le logement avec un diamètre intérieur et peut être ancré ensuite par une rotation.

11. Implant de la colonne vertébrale selon l'une des revendications 1 à 8, **caractérisé en ce que** l'élément de liaison (13) présente une cavité (14) autour de l'axe longitudinal, qui facilite la déformation élastique pour l'introduction dans le logement.

12. Implant de la colonne vertébrale selon la revendication 11, **caractérisé en ce qu'**il comprend un pivot (15) qui peut être inséré dans la cavité (14) après l'introduction de l'élément de liaison (13) dans le logement (4).

13. Implant de la colonne vertébrale selon l'une des revendications 1 à 12, **caractérisé en ce que** l'élément de liaison peut être serré (17) et l'élément de liaison (5) peut être serré après l'introduction (17a).

14. Implant de la colonne vertébrale selon l'une des revendications 1 à 12, **caractérisé en ce que** l'élément de liaison présente un dispositif formant crochet (19) dans lequel peut être accrochée une clavette (20) sous précontrainte de l'élément de liaison (5).

15. Implant de la colonne vertébrale selon l'une des revendications 6 à 14, **caractérisé en ce que** les nervures présentent sur l'élément de liaison un plan incliné, le logement présente sur la tête de vis les mêmes nervures avec un plan incliné (22) et agit ainsi comme un écrou de sorte que l'élément de liaison peut être vissé et guidé par rotation autour de l'axe longitudinal par les têtes de vis.
